# EUROPEAN PATENT SPECIFICATION

(11) **EP 2 455 948 B1**
(45) Date of publication and mention of the grant of the patent: **08.06.2016**
(21) Application number: 10799734.8
(22) Date of filing: 30.06.2010
(51) Int. Cl.: H01B 7/00, G02B 23/24, H01R 9/05, H01R 11/01

(54) **CABLE ASSEMBLY**
KABELANORDNUNG
CONFIGURATION DE CABLES

(30) Priority: 13.07.2009 JP 2009164883
(43) Date of publication of application: 23.05.2012
(73) Proprietor: Olympus Corporation, Shibuya-ku Tokyo 151-0072 (JP)
(72) Inventor: SEKIDO, Takanori, Tokyo 151-0072 (JP); YOSHIZAWA, Fukashi, Tokyo 151-0072 (JP)
(74) Representative: Winter, Brandl, Fürniss, Hübner, Röss, Kaiser, Polte - Partnerschaft mbB
(86) International application number: PCT/JP2010/061207
(87) International publication number: WO 2011/007674

(56) References cited:
- WO-A1-2004/047509
- JP-A- 4 109 511
- JP-A- 7 029 423
- JP-A- H04 179 074
- JP-A- H11 295 617
- JP-A- 2000 278 837
- JP-A- 2001 014 956
- JP-A- 2003 178 826
- JP-A- 2004 006 113
- JP-A- 2006 127 790
- JP-A- 2007 278 810
- JP-A- 2007 335 174
- JP-B2- 3 863 583
- US-A- 5 281 762
- US-B2- 6 958 450

## Description

The present invention relates to a cable assembly that can set a plurality of cables in contact as one bundle.

Conventionally (see for example JP 3863583 A), a cable assembly is used in a distal-end circuit of an endoscope to set two or more cables in contact as one bundle. A small-sized cable assembly is implemented by shortening the length of a hard section that functions as a connecting terminal section. Cable assemblies have a plurality of cables, with the cables being fixed together by using an array block that forms a hard section, and they have a cable connecting end surface that is formed by polishing the distal ends of the fixed cables in such a manner that the distal ends together form the same plane. The cables are set in contact by using an anisotropic conductive adhesive film (ACF: Anisotropic Conductive Adhesive Film) or an anisotropic conductive adhesive paste (ACP: Anisotropic Conductive Adhesive Paste), which is applied to the cable connecting end surface. Thereby, the cables are set in contact as one bundle and the length of the hard section is set short.

Document JP 3863583 A hence discloses a cable assembly comprising: a cable group, in which a plurality of cables is bound together, each out of the plurality of cables having at least a cable core and an internal insulator formed on an outer circumference of the cable core as well as a shield wire formed on an outer circumference of the internal insulator and an external insulator formed on an outer circumference of the shield wire; and a cable fixing member for binding the plurality of cables together into the cable group; wherein each of the cable cores and the shield wires comprises a connecting end surface, the connecting end surface being formed by cutting the cable group fixed by the cable fixing member along a cutting plane and by polishing the cutting plane that leads to the cable group.

The above-mentioned conventional cable assembly uses an array block to fix cables to each other; however, even after the above-mentioned polishing process, because misalignment may occur between the cable and the array block or, if the cables are coaxial cables or the like, misalignment may occur between an external insulator and a shield wire or between an internal insulator and a cable core, it is difficult to arrange each cable end in the same plane. If such a cable assembly is connected directly to a substrate or the like, some cables will be in poor contact.

JP 2001 014956 A describes a connecting cable for establishing an electrical connection between integrated circuit elements. A plurality of individual wires is bundled by a fixing member to a cable assembly and at the respective opposite terminal ends of the cable assembly conductor pads are applied to the free faces of the wires germinating there. These conductor pads project beyond the terminal ends of the cable assembly and are for making contact with respective connecting terminals at the circuit elements' sides.

JP 2001 014956 describes a cable connecting structure being substantially similar to that of JP 2001 014956 A, however without conductor pads applied to the free ends of the individual wires. Instead, elastic conductors protrude from a circuit substrate and are for contact with the respective free ends of the wires. A sleeve is a fixing member for bundling the individual wires and for establishing a contact pressure between the elastic conductors of the substrate and the associated free ends or faces of the wires.

Other conventional cable assemblies are known from e.g. JP 2000 278837 A, JP 2007 335 174 A and JP 2003 178826 A.

Both JP 2001 014956 and JP 2001 014956 suffer from the drawback that there is a concern that, due to a bending of the individual wires, which are not fixed together by the fixing member, or a change in temperature, etc., the individual wires might move and are pulled in a direction away from the correct contact position.

The present invention has been made in view of the above, and it is an object of the present invention to provide a small-sized cable assembly that can set each cable in good contact.

According to the present invention there is provided a cable assembly comprising: a cable group, in which a plurality of cables is bound together, each out of the plurality of cables having at least a cable core and an internal insulator formed on an outer circumference of the cable core as well as a shield wire formed on an outer circumference of the internal insulator and an external insulator formed on an outer circumference of the shield wire; a cable fixing member for binding the plurality of cables together into the cable group; conductor layers, which are provided corresponding to a cross section of each of the cable cores and the shield wires on a connecting end surface, the connecting end surface being formed by cutting the cable group fixed by the cable fixing member along a cutting plane and by polishing the cutting plane that leads to the cable group, wherein a width of the respective conductor layers exceeds a width of the cross section of the cable cores and a width of the cross section of the shield wires, so that a part of the respective conductor layers is provided on an area corresponding to the insulator on the outer circumference of cable core or shield wire, so that upon provision of the conductor layers corresponding to said cross section of each of the cable cores and the shield wires said conductor layers function as stoppers preventing a movement of the cable cores and shield wires and thus of the respective cables inwardly with respect to the cable fixing member.

Generally, the conductor layer functions as a stopper that prevents the cable core from moving inwardly in a direction away from contact with the respective counter-contact or substrate pattern.

Preferably the cable fixing member is a resin that at least encases an outer periphery of the cable gruop .

In the cable assembly according to the present invention as set forth in the invention described above, the conductor layers can cover the respective surfaces of the connecting ends such that the conductor layers protrude out from the respective connecting ends.

In the cable assembly according to the present invention as set forth in the invention described above, each of the conductor layers may be shaped like a ring and is over the surface of the connecting end and an insulating section that is adjacent to the connecting end.

In the cable assembly according to the present invention as set forth in the invention described above, each cable may have a common shield wire, and the conductor layers connect surfaces of connecting ends of the shield wires to each other.

In the cable assembly according to the present invention as set forth in the invention described above, a connecting conductive member may be formed on each of the conductor layers to connect the connecting end to an external connecting end that corresponds to the connecting end.

In the cable assembly according to the present invention as set forth in the invention described above, an insulator film may be formed on an area other than an area on which the connecting conductive member is formed.

According to the present invention, in the cable assembly having a plurality of cables fixed together by using a cable fixing member and formed with a connecting end surface that includes thereon connecting ends of the cables, conductor layers are provided to cover surfaces of the connecting ends that are on the connecting end surface. The conductor layer bonds a connecting end and an insulating section together. Therefore, a connecting end cannot move inwardly relative to the insulating section, which is adjacent to the connecting end, and the connecting end is always positioned on the connecting end surface. Accordingly, reliable electrical connection to an external connecting terminal can be made, and a small-sized cable assembly can be made while keeping the reliable connection of a plurality of cables.

The invention now will be described by reference to accompanying drawings, in which:
FIG. 1 is an explanatory diagram that explains production of a cable assembly according to the present invention;
FIG. 2 is an explanatory diagram that explains a process of connecting the cable assembly to an external substrate;
FIG. 3 is a cross-sectional view of a state of a conductor layer that is near the cable-assembly end surface;
FIG. 4 is a cross-sectional view of a cable assembly according to an embodiment of the present invention;
FIG. 5 is a cross-sectional view of a modification of the conductor layer;
FIG. 6 is a cross-sectional view of a modification of the conductor layer that covers a shield wire;
FIG. 7 is a cross-sectional view of a modification of the conductor layer that covers a shield wire;
FIG. 8 is a cross-sectional view of a modification in which a connecting conductive member is provided;
FIG. 9 is a cross-sectional view of a modification in which a connecting conductive member and an insulating film are provided; and
FIG. 10 is a cross-sectional view of a modification in which a conductive projection and an insulator film are provided.

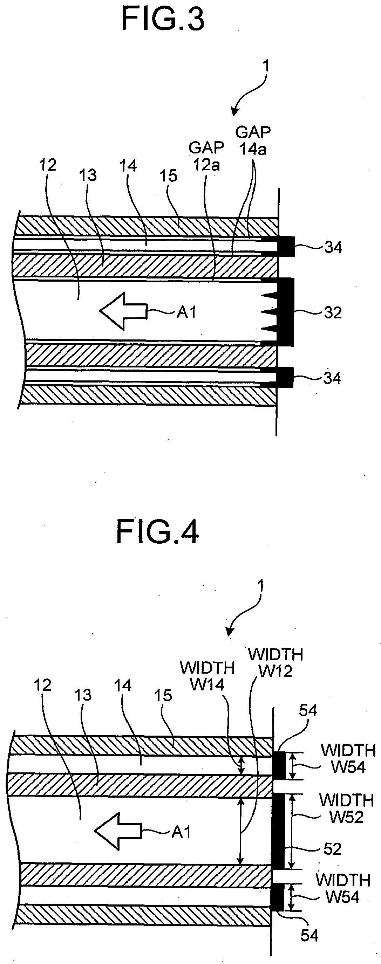

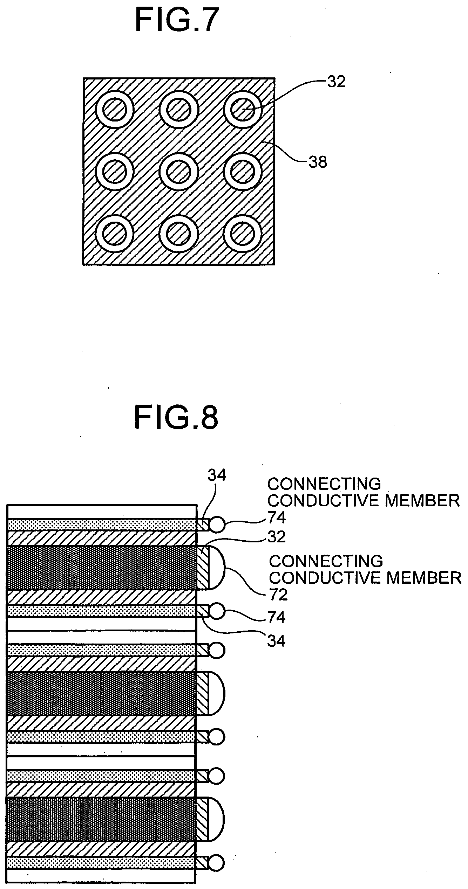

An exemplary embodiment of the present invention is described in detail below with reference to the accompanying drawings. It should be noted that the present invention has its focus on the embodiment depicted in Fig. 4. Especially the embodiments according to Figs. 8 to 10 do not fall under the invention as claimed, are, however, shown and described for the purpose of explanation.

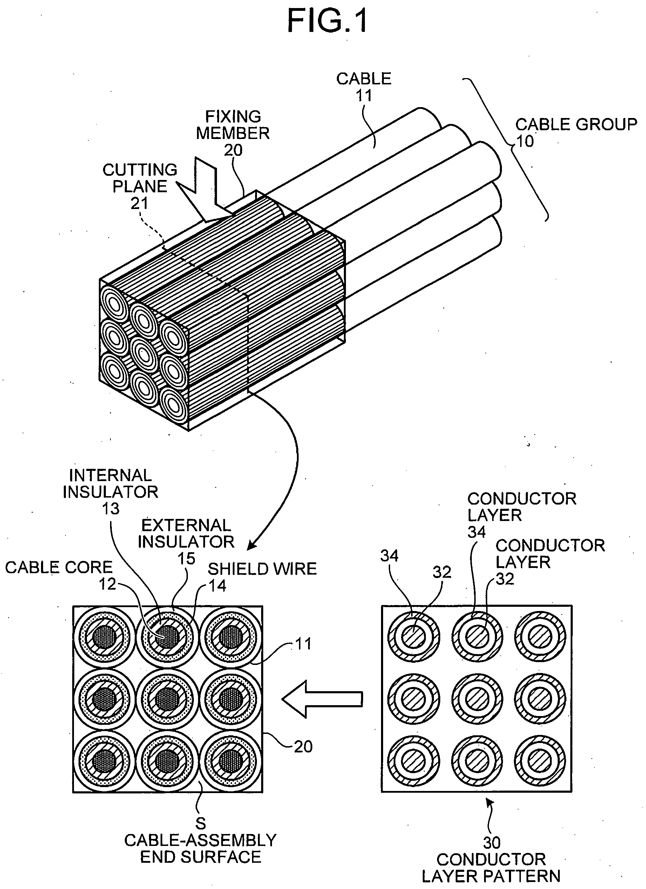

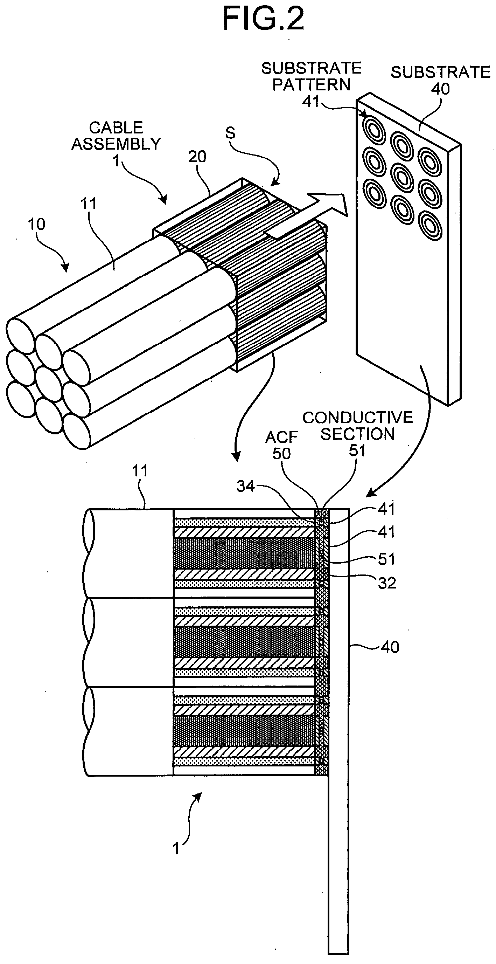

FIG. 1 is an explanatory diagram that explains production of a cable assembly according to the present invention. FIG. 2 is an explanatory diagram that illustrates an application of the produced cable assembly. As illustrated in FIG. 1, in a cable assembly, a plurality of cables 11 are bound together, thereby forming a cable group 10, and the cables 11 of the cable group 10 are fixed to each other by using a fixing member 20 that is made of a resin, etc. In this fixed state, in order to form a cable-assembly end surface S that is a connecting end surface of the cable assembly, part of the fixing member 20 is cut along a cutting plane 21. In the cut surface, a connecting end surface, which leads to the cable group 10 is then polished so that the formed connecting end surface includes the connecting end of each of the cables 11 on the same plane.

Each of the cables 11 is a coaxial cable having the same diameter. A shield wire 14 is formed on an internal insulator 13, surrounding the outer circumference of a cable core 12. An external insulator 15 is formed on the outer circumference of the shield wire 14. Although, in FIG. 1, nine (3x3) cables 11 are adjacently arranged in parallel in such a manner that the cross section is substantially a rectangular, it is allowable to use an array block in which cable insertion holes are formed in advance. If so, the cables 11 are not adjacently arranged. It is preferable to perform cutting and polishing involving the array block.

When the cable-assembly end surface S is formed by the polishing process, corresponding conductor layers 32 and 34 in a conductor layer pattern 30 are formed on the connecting end surfaces, which are cross sections of the cable core 12 and the shield wire 14 of each cable 11. The conductor layers 32 and 34 are implemented by metallic films, and the conductor layer pattern 30 is formed by electrolytic plating, non-electrolytic plating, or sputtering. The conductor layers 32 and 34 can have either a single-layer structure or a multilayer structure. A multilayer structure having a Ni/Au multilayer film, in which an Au layer is top layer and a Ni layer is under layer, is preferable because a strong bonding to a connecting end surface is formed. By using the Ni/Au multilayer film, various bonding methods become applicable, not only ACF and ACP bonding but also solder bump bonding and Au bump bonding, thereby increasing the flexibility in bonding.

A thus formed cable assembly 1 is connected, as illustrated in FIG. 2, to a substrate 40 on which a substrate pattern 41 is formed that corresponds to the conductor layer pattern 30 of the cable-assembly end surface S of the cable assembly 1. An anisotropic conductive resin material, such as an ACF 50, is inserted between the cable assembly 1 and the substrate 40. By thermal compression bonding using the anisotropic conductive resin material, a conductive section 51 is formed on a compressed area, and the cable assembly 1 and the substrate 40 are bonded together. Because the areas where the conductor layers 32 and 34 are formed are convex and are protruding outward from the cable-assembly end surface S, the areas are under a high pressure and thus the conductive section 51 is formed via a dense filler, etc., within the ACF 50.

The shapes of the conductor layers 32 and 34 may be the same as the shapes of the cross sections of the cable core 12 and the shield wire 14, respectively. As illustrated in FIG. 3, the actual cables 11 have a gap 12a between the cable core 12 and the internal insulator 13 and a gap 14a between the shield wire 14 and either the internal insulator 13 or the external insulator 15. The conductor layers 32 and 34 are formed to be inserted in the gaps 12a and 14a, respectively, near the cable-assembly end surface S. In other words, the conductor layers 32 and 34 are formed to be inserted in the gaps 12a and 14a, respectively, like wedges. With a stress that occurs in a line-width direction near the cable-assembly end surface S, the cable core 12 and the internal insulator 13, the shield wire 14 and the internal insulator 13, and the shield wire 14 and the external insulator 15 are fixed together. In general, there is a concern that, due to a bending of the cables 11, which are not fixed together by the fixing member 20, or a change in temperature, etc., the cable core 12 and the shield wire 14 are pulled toward a direction indicated by an arrow A1; however, the above-mentioned fixing prevents the above problem, and connecting end surfaces are reliably formed on the cable-assembly end surface S. In other words, the conductor layers 32 and 34 function as stoppers that prevent the cable core 12 and the shield wire 14 from moving inwardly in the direction indicated by the arrow A1. If the cable core 12 and the shield wire 14 are formed by a plurality of wires, such as twisted wires, the conductor layers 32 and 34 are inserted between wires like wedges near the cable-assembly end surface S. A stress occurring in the line-width direction makes them function as stoppers.

According to the present invention and as illustrated in FIG. 4, overlapping conductor layers 52 and 54 may be applied, the widths of which exceed the width W12 of the cable core 12 and the width W14 of the shield wire 14, respectively. That is, a width W52 of the conductor layer 52 satisfies W52>W12 and a width W54 of the conductor layer 54 satisfies W54>W14. In this case, parts of the conductor layers that protrude from the areas of the cable core 12 and the shield wire 14 prevent the cable core 12 and the shield wire 14 from moving inwardly in the A1 direction, and at the same time, the conductor layers 52 and 54 are conductively connected to the cable core 12 and the shield wire 14, respectively, on the cable-assembly end surface S.

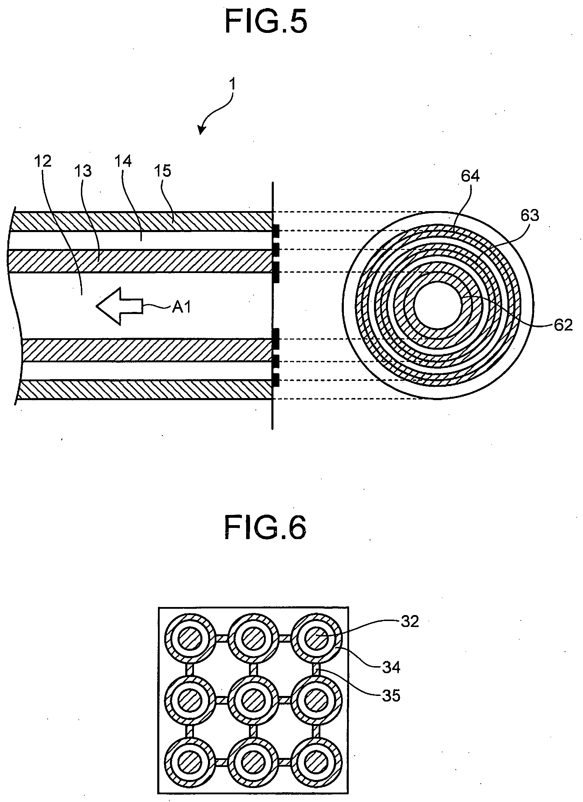

Also, as illustrated in FIG. 5, ring-like shaped conductor layers 62, 63, 64 may be applied, which are only formed near an area at which the cable core 12 and the internal insulator 13 are in contact, near an area at which the shield wire 14 and the internal insulator 13 are in contact, and near an area at which the shield wire 14 and the external insulator 15 are in contact. Although the conductor area is reduced with this arrangement, inward movement of the cable core 12 and the shield wire 14 is prevented.

Furthermore, as illustrated in FIG. 6, in the case in which the shield wire 14 of each of the cables 11 is a shield wire having a common potential, the conductor layers 34 may be connected to each other via a conductor layer 35. This facilitates connection of the shield wires 14 and reduces contact noises more effectively. This, especially, allows a shield area on the side of an external connecting terminal to be formed at one position.

Similarly, as illustrated in FIG. 7, the areas between the adjacent shield wires 14 may be covered with a conductor layer 38. In other words, the entire area on the cable-assembly end surface S other than the areas of the cable core 12 and the internal insulator 13 may be covered with the conductor layer 38.

Also, as illustrated in FIG. 8, connecting conductive members 72 and 74, which are solder projections, may be further formed on the conductor layers 32 and 34, respectively. The solder projections are formed by applying solder pastes or solder powders to the surfaces of the connecting ends of the cable core 12 and the shield wire 14, and then fusing the solder by reflow. Because the solder projections can be directly used as connecting members that connect the cable assembly 1 to the external substrate 40, this simplifies a connecting-material supplying process in connecting the cable assembly 1 to the substrate 40.

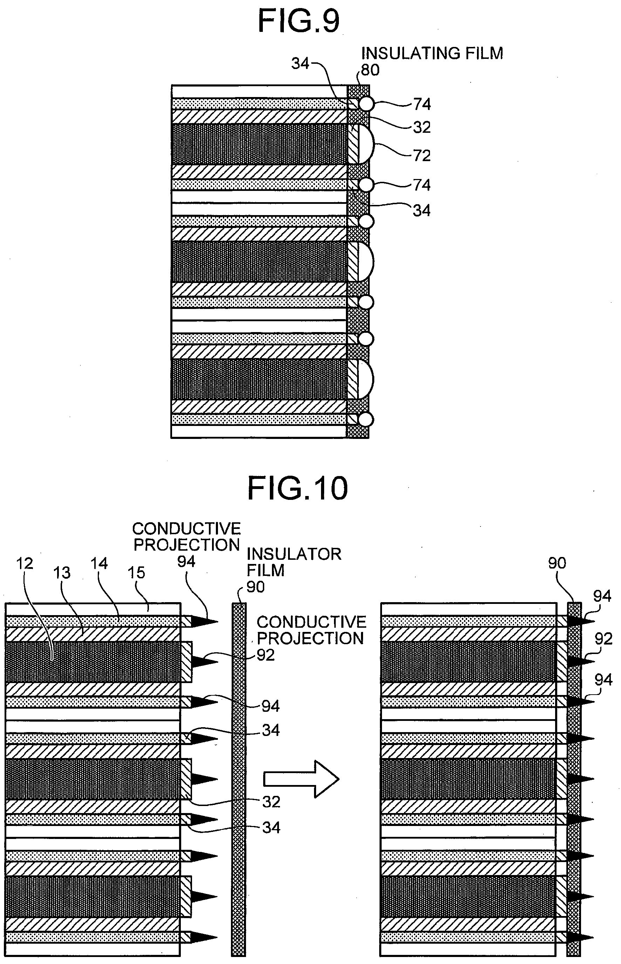

Furthermore, as illustrated in FIG. 9, a resist may be applied between the connecting conductive members 72, 74 and the conductor films 32, 34 as an insulating film 80. In this case, the thickness of the insulating film 80 is such that the distal ends of the connecting conductive members 72 and 74 are exposed. With this, a short-circuit between the cable core 12 and the shield wire 14 can be prevented.

Also, as illustrated in FIG. 10, conductive projections 92 and 94 may be formed on the conductor layers 32 and 34 of the connecting end surfaces of the cable core 12 and the shield wire 14, respectively, by supplying an Ag paste to form projections and then hardening the projections by a heat. Furthermore, the cable-assembly end surface S is covered with an insulator film 90 and cause the conductive projections 92 and 94 to pierce through the insulator film 90 so that the distal ends of the conductive projections 92 and 94 are exposed on the surface of the insulator film 90. With this, a short-circuit between the conductive projections 92 and 94 is prevented. Because the insulator film 90 is especially used in this case, an applying process of insulator, etc., is unnecessary; therefore, an insulating process can be performed easily.

Reference Signs List
- 1: CABLE ASSEMBLY
- 10: CABLE GROUP
- 11: CABLE
- 12: CABLE CORE
- 12a, 14a: GAP
- 13: INTERNAL INSULATOR 14 SHIELD WIRE
- 15: EXTERNAL INSULATOR
- 20: FIXING MEMBER
- 21: CUTTING PLANE
- 30: CONDUCTOR LAYER PATTERN
- 32, 34, 35, 38, 52, 54, 62, 63, 64: CONDUCTOR LAYER
- 40: SUBSTRATE
- 41: SUBSTRATE PATTERN
- 50: ACF
- 51: CONDUCTIVE SECTION
- 72, 74: CONNECTING CONDUCTIVE MEMBER
- 80: INSULATING FILM
- 90: INSULATOR FILM
- 92, 94: CONDUCTIVE PROJECTION
- S: CABLE-ASSEMBLY END SURFACE

## Claims

1. A cable assembly (1) comprising:
a cable group (10), in which a plurality of cables (11) is bound together, each out of the plurality of cables (11) having at least a cable core (12) and an internal insulator (13) formed on an outer circumference of the cable core (12) as well as a shield wire (14) formed on an outer circumference of the internal insulator (13) and an external insulator (15) formed on an outer circumference of the shield wire (14);
a cable fixing member (20) for binding the plurality of cables (11) together into the cable group (10);
conductor layers (52, 54), which are provided corresponding to a cross section of each of the cable cores (12) and the shield wires (14) on a connecting end surface, the connecting end surface being formed by cutting the cable group (10) fixed by the cable fixing member (20) along a cutting plane (21) and by polishing the cutting plane (21) that leads to the cable group (10),
wherein a width of the respective conductor layers (52, 54) exceeds a width (W12) of the cross section of the cable cores (12) and a width (W14) of the cross section of the shield wires (14), so that a part of the respective conductor layers (52, 54) is provided on an area corresponding to the insulator (13, 15) on the outer circumference of cable core (12) or shield wire (14),
so that upon provision of the conductor layers (52, 54) corresponding to said cross section of each of the cable cores (12) and the shield wires (14) said conductor layers (52, 54) function as stoppers preventing a movement of the cable cores (12) and shield wires (14) and thus of the respective cables (11) inwardly with respect to the cable fixing member (20).

2. The cable assembly (1) according to claim 1, wherein the cable fixing member (20) is a resin that at least encases an outer periphery of the cable group (10).

## Patentansprüche

1. Kabelanordnung (1), aufweisend:
eine Kabelgruppe (10), in welcher eine Mehrzahl von Kabeln (11) zusammengebündelt ist, wobei jedes aus der Mehrzahl von Kabeln (11) wenigstens einen Kabelkern (12) und einen Innenisolator (13), der an einem Außenumfang des Kabelkerns (12) ausgebildet ist, sowie einen Abschirmdraht (14), der an einem Außenumfang des Innenisolators (13) ausgebildet ist und einen Außenisolator (15) aufweist, der an einem Außenumfang des Abschirmdrahts (14) ausgebildet ist;
ein Kabelbefestigungsteil (20), um die Mehrzahl von Kabeln (11) zu der Kabelgruppe (10) zusammenzubündeln;
Leiterschichten (52, 54), welche entsprechend einem Querschnitt eines jeden der Kabelkerne (12) und der Abschirmdrähte (14) an einer Verbindungsendfläche angeordnet sind, wobei die Verbindungsendfläche gebildet wird durch Schneiden der Kabelgruppe (10), die durch das Kabelbefestigungsteil (20) festgelegt ist, entlang einer Schnittebene (21) und durch Polieren der Schnittebene (21), was zu der Kabelgruppe (10) führt,
wobei eine Breite der jeweiligen Leiterschichten (52, 54) eine Breite (W12) des Querschnitts der Kabelkerne (12) und eine Breite (W14) des Querschnitts der Abschirmdrähte (14) übersteigt, so dass ein Teil der jeweiligen Leiterschichten (52, 54) an einer Fläche entsprechend dem Isolator (13, 15) an dem Außenumfang des Kabelkerns (12) oder des Abschirmdrahts (14) angeordnet ist,
so dass bei Bereitstellung der Leiterschichten (52, 54) entsprechend dem Querschnitt eines jeden der Kabelkerne (12) und der Abschirmdrähte (14) die Leiterschichten (52, 54) als Anschläge dienen, welche eine Bewegung der Kabelkerne (12) und der Abschirmdrähte (14) und damit der jeweiligen Kabel (11) bezüglich des Kabelbefestigungsteils (20) nach innen verhindern.

2. Kabelanordnung (1) nach Anspruch 1, wobei das Kabelbefestigungsteil (20) ein Harz ist, welches zumindest einen Außenumfang der Kabelgruppe (10) einschließt.

## Revendications

1. Ensemble à câbles (1), comprenant :
un groupe de câbles (10), dans lequel une pluralité de câbles (11) est liée ensemble, chacun parmi la pluralité de câbles (11) possédant au moins une âme de câble (12) et un isolant interne (13) formé sur une circonférence extérieure de l'âme de câble (12) ainsi qu'un fil de garde (14) formé sur une circonférence extérieure de l'isolant interne (13) et un isolant externe (15) formé sur une circonférence extérieure du fil de garde (14) ;
un organe de fixation de câble (20) pour lier la pluralité de câbles (11) ensemble dans le groupe de câbles (10) ;
des couches conductrices (52, 54), qui sont prévues de façon correspondante à une section transversale de chacune des âmes de câble (12) et de chacun des fils de garde (14) sur une surface d'extrémité de connexion, la surface d'extrémité de connexion étant formée en coupant le groupe de câbles (10) fixé par l'organe de fixation de câble (20) le long d'un plan de coupe (21) et en polissant le plan de coupe (21) qui conduit au groupe de câbles (10),
dans lequel une largeur des couches conductrices respectives (52, 54) excède une largeur (W12) de la section transversale des âmes de câble (12) et une largeur (W14) de la section transversale des fils de garde (14), pour qu'une partie des couches conductrices respectives (52, 54) soit prévue sur une zone correspondant à l'isolant (13, 15) sur la circonférence extérieure de l'âme de câble (12) ou du fil de garde (14),
pour que, lors de la fourniture des couches conductrices (52, 54) correspondant à ladite section transversale de chacune des âmes de câble (12) et de chacun des fils de garde (14), lesdites couches conductrices (52, 54) servent de butées empêchant un mouvement des âmes de câble (12) et des fils de garde (14) et ainsi des câbles respectifs (11) vers l'intérieur par rapport à l'organe de fixation de câble (20).

2. Ensemble à câbles (1) selon la revendication 1, dans lequel l'organe de fixation de câble (20) est une résine qui enferme au moins une périphérie extérieure du groupe de câbles (10).
